Europäisches Patentamt

(19) European Patent Office

Office européen des brevets

(11) Publication number : **0 470 855 B1**

## (12) EUROPEAN PATENT SPECIFICATION

(45) Date of publication of patent specification :
27.07.94 Bulletin 94/30

(51) Int. Cl.[5] : **A23L 1/0534,** A23L 1/308,
A23L 1/164, A23L 1/18

(21) Application number : **91307350.8**

(22) Date of filing : **09.08.91**

(54) **Use of hydrolyzed cellulose derivatives in snack foods.**

(30) Priority : **09.08.90 GB 9017452**

(43) Date of publication of application :
**12.02.92 Bulletin 92/07**

(45) Publication of the grant of the patent :
**27.07.94 Bulletin 94/30**

(84) Designated Contracting States :
**AT BE CH DE DK ES FR GB GR IT LI LU NL SE**

(56) References cited :
**EP-A- 0 228 951
DE-B- 1 270 385
FR-A- 2 218 882
US-A- 3 023 104
US-A- 3 397 198
US-A- 4 517 204
US-A- 4 701 445**

(73) Proprietor : **Alko Ltd.
P.O. Box 350
SF-00101 Helsinki (FI)**

(72) Inventor : **Lindley, Michael
17 Highway,
Edgcombe Park
Crowthorne, Berks (GB)**
Inventor : **Bagley, Lindsey
4 Barn Close
Maidenhead, Berks (GB)**
Inventor : **Bosdet, Sarah
45 All Saints Close
Glebe Park, Wokingham, Berks (GB)**

(74) Representative : **Collier, Jeremy Austin Grey et al
J.A.KEMP & CO.
14 South Square
Gray's Inn
London WC1R 5LX (GB)**

## Description

This invention relates to novel foodstuff formulations comprising degradation products of cellulose derivatives having little or no caloric value.

In our European Patent Application no. 90301463.7 (publication no. 0382578) we have described and claimed foodstuff compositions comprising a mixture of oligomers derived from degradation of a cellulose derivative substituted for at least a portion of a high calorie ingredient of the foodstuff composition, the said mixture of oligomers having an average degree of polymerisation in the range of 3 to 300, and the average molecular weight in the range of 500 to 100,000 determined on the basis of intrinsic viscosity. It is stated in the said application that the high calorie ingredient is preferably a fat and/or a carbohydrate and the specific foodstuffs compositions described are cake compositions comprising a fat ingredient, a sweetener, a flour, a raising agent, an egg ingredient and water; an icing composition comprising a fat ingredient, a sweetener and water; a mayonnaise composition comprising a fat ingredient, a thickener, an acidifier, an emulsifier and water; and a spread composition comprising a fat ingredient, an emulsifier and/or a stabiliser and water.

We have now discovered that the aforesaid mixture of oligomers derived from degradation of a cellulose derivative are particularly advantageously incorporated into food products based on gelled starch. A gelled starch food product is a food product in which the main ingredient is starch (present as such or in the form of a high starch flour) which is mixed with water to produce a gel which is then hardened by heating (i.e. cooked) to provide the main structural matrix of the food product, which does not depend for its structural integrity on the presence on any protein such as wheat gluten, casein or egg protein. The invention is especially advantageously applied to the production of gelled starch products which are cooked at elevated temperature during extrusion, i.e. under pressure, or by frying, i.e. immersion (partial or complete) in fat at high temperature. When used in such products the aforesaid oligomer mixtures are capable of conferring unique and attractive textures which can be achieved with less frying and/or less fat than has heretofore been thought necessary.

The present invention accordingly provides gelled starch food products (as hereinbefore defined) comprising from 0.05 to 10% by weight of the starch of a mixture of oligomers derived from the degradation of a cellulose derivative, the said mixture of oligomers having an average degree of polymerisation in the range of 3 to 500, usually 3 to 300 and an average molecular weight in the range of 500 to 100,000, preferably 500 to 60,000, determined on the basis of intrinsic viscosity.

The soluble cellulose derivative from which the mixture of oligomers is obtained is preferably carboxymethylcellulose, methyl-cellulose, methylethylcellulose, hydroxypropylmethylcellulose, hydroxypropylcellulose or a mixture thereof.

Preferably the cellulose derivative starting material has a degree of substitution of between about 0.1 to about 3.0. "Degree of substitution" refers to the number of derivative groups (e.g. carboxymethyl, hydroxypropyl) per monomer units in the cellulose backbone (branched or straight chain). A degree of substitution of 0.2 means, for example, that there is about one derivative substituent per every 5 monomer units in the cellulose derivative. A degree of substitution of three (3) would mean there are three derivative substituents per every monomer unit in the cellulose derivative.

The cellulose derivative may be degraded by enzymatic, chemical or physical agents/mechanisms. Where an enzyme preparation is utilized, the enzyme preparation is typically selected from the group of cellulases, modified cellulases and mixtures thereof.

Where degradation of the cellulose derivative is effected by chemical or physical means, chemical hydrolysis, chemical oxidation and physical depolymerization are preferred mechanisms for achieving the desire oligomeric mixtures used in the invention.

An enzyme preparation may be a cellulase or modified cellulase (i.e., modified by removing or preventing the formation of mono- and disaccharides producing enzymes in the cellulase preparation in the first instance, e.g., by genetic alteration of the microorganism from which the cellulase preparation is prepared) preferably produced from microorganisms selected from the group of Trichoderma, Aspergillus and Penicillium. Most preferably a cellulase preparation is derived from Trichoderma reesei from which at least one of beta-glucosidase and cellobiohydrolase activities have been removed. An enzyme preparation most preferably comprises endo-1,4-beta-glucanase.

The cellulolytic agent may be a hydrolytic chemical or mixture of chemicals such as a hydrolytic acid or base treatment solution (e.g. containing $H_2SO_4$, HC1, NaOH or $NH_4OH$) or an oxidative chemical or chemical solution (e.g. solutions containing oxygen, hydrogen peroxide, ozone or mixtures thereof).

The cellulose derivative may be hydrolyzed by treating the cellulose derivative with a solution of acid or base. Typical acid treatment solutions might contain sulphuric acid, hydrochloric acid, phosphoric acid, nitric acid or mixtures of two or more of the foregoing. Typical base solutions might contain a hydroxide ion containing or producing material such as an alkali hydroxide (e.g. sodium hydroxide), ammonium hydroxide, and mixtures of two or more of the foregoing. The concentration of the acid or base in the treatment solution and the treatment time and temperature may vary depending on the degree of degradation of the cellulose derivative which is desired. The person skilled in the art will recognize that higher acid or base concentrations, treatment time and treatment temperatures will generally result in a higher degree of degradation of the cellulose derivative (i.e. an oligomeric product mixture having a lower average DP and molecular weight). And, lower acid or base concentrations and treatment times and temperature will generally produce oligomeric product mixtures of higher average DP and molecular weight. In any event where an acid or base hydrolysis treatment is utilized, the acid or base concentration and the treatment time and temperature is selected to produce a mixture of oligomers having an average DP of preferably between 3 and 300, especially 5 to 100 and even more preferably 5 to 50, an average molecular weight of between 500 and 100,000 and which most preferably contains less than about 25% by weight of mono- and disaccharides such as glucose and cellobiose.

The selected cellulose derivative may also be degraded by oxidation with such agents as oxygen or hydrogen peroxide in basic solution or with ozone. Such oxidative treatments and reaction conditions are well known in the art. Gaseous agents such as oxygen or ozone would typically be bubbled continuously through the solution for a suitable time and at a suitable temperature. An oxidative treatment with peroxide might comprise treating a selected cellulose derivative with a solution of hydrogen peroxide of suitable concentration and at a suitable temperature.

The oligomeric mixtures may also be produced by physical (mechanical) depolymerization methods such as by subjecting a solution of a selected cellulose derivative to treatment with relatively high frequency sound waves with a sonicator. Other physical treatments well known in the art such as chopping or shearing a selected cellulose derivative with, for example, a high speed mixer or homogenizer may be employed to effect depolymerization.

Whatever conventional chemical (hydrolytic, oxidative or otherwise) or physical treatments are employed, the conditions and the degree of treatment are selected such that the oligomeric mixture resulting from the initial treatment has an average DP of between 3 and 500, an average molecular weight of between 500 and 100,000 and preferably contains less than about 25% by weight of mono- and disaccharides and most preferably less than about 10% by weight of mono- and disaccharides.

Enzymes which may be used in this invention are various food-grade cellulase preparations. They can be produced from a multitude of different microorganisms such as strains of Trichoderma, Aspergillus, Penicillium, etc. A selected microorganism strain is grown by conventional means in a medium containing food grade materials such that the cellulases are produced, the microorganism is separated from the medium, the medium is collected and typically concentrated and dried. These enzymes can be used as such or in mixtures and they can be modified in many different ways known to the man skilled in the art. A most preferred enzyme preparation is produced from Trichoderma reesei, from which preparations the beta-glucosidase and/or the cellobiohydrolase activities are removed chromatographically or genetically. Beta-glucosidase and/or cellobiohydrolase activities are preferably removed from the selected cellulase preparation so as to prevent the degradation of the cellulose derivative into mono- and disaccharides. Genetic alteration of the appropriate enzyme producing microorganism may be effected with radiation or mutagenic chemical agents (or by gene inactivation by recombinant DNA methods) so as to disenable production of beta-glucosidase and cellobiohydrolase by the microorganism. Cellulase preparations suitable for use herein are e.g. the commercially available cellulase preparations designated as the Econase series as produced by Alko Ltd., Helsinki Finland.

The cellulose derivative hydrolysates may be prepared from soluble cellulose derivatives as defined above by an enzymatic hydrolysis utilizing a cellulase preparation having endo-1,4-beta-glucanase as the sole active hydrolytic agent such that only insignificant amounts of mono- and disaccharides which are absorbed in human intestine (e.g., glucose) or hydrolyzed by the intestinal bacterial flora (e.g., cellobiose), are produced. On the other hand the average degree of polymerization (DP) of the oligomers formed by such a hydrolysis is lower than 500, and thus the viscosity of solutions of the hydrolysate is reduced significantly compared to the viscosity of solutions of the unhydrolysed cellulose derivatives. The specific conditions suitable for and the specific time sufficient to secure the desired hydrolysis may be readily determined for each selected cellulose derivative and each selected enzyme preparation.

Similarly where degradation is carried out using chemical or physical means, the average DP of the oligomers is less than 500 and the viscosity of the resulting mixture is significantly reduced. Most preferably in such embodiments, the treatment conditions are selected such that the resulting oligomeric mixtures contain less than about 5% by weight of mono- and disaccharides.

The degraded cellulose derivative products used in the invention (and fractions thereof) dissolve rapidly in cold and hot water and are physiologically inert. The cellulose derivatives used in the present invention are as such non-caloric. Because a degradative treatment according to the present invention does not produce significant amounts of metabolizable sugars, the resulting oligomeric mixtures used in this invention with their improved properties, are especially useful as low-caloric ingredients in food stuffs.

By conventional means an initially degraded cellulose derivative mixture may be further separated into fractions of oligomers of differing average chain lengths. The viscosity of the various fractions will vary with the degree of average chain length of the oligomers contained within a fraction. Depending on the particular food stuff application, the invention further contemplates selecting one or more fractions from an initial oligomeric mixture having a viscosity (average chain length) which is most appropriate for the particular food stuff application. The selection of a particular average chain length fraction and the amount of such a fraction to be used in any given food stuff application may vary, it being recognized that the higher the absolute amount of the fraction desired to be used in a particular foodstuff, the lower the viscosity (average molecular weight) of the fraction of mixture of oligomers should be used.

It is to be further recognized that an oligomeric mixture falling within a particular range of viscosities may be preferred in any particular food recipe insofar as it may be desirable to obtain an end product which resembles the eating quality of the normal recipe containing the normal relatively high level of fat or other high calorie ingredients.

The following Examples 1-4 set forth typical exemplary routines for preparing a cellulase and various cellulose derivative hydrolysates therefrom.

Example 1 -- Typical Cellulase Preparation

The beta-glucosidase activity was removed by ion exchange chromatography from the commercially available cellulose preparation, Econase CE, as so designated by Alko Ltd., Helsinki, Finland which was produced from a strain of Trichoderma reesei. The cellulase preparation (column A, Table 1) was passed through a cation exchange column (S-Sepharose FF, Pharmacia, LKB Biotechnology AB, Uppsala, Sweden) which was equilibrated with 50mM sodium acetate pH 3.8 equilibrium buffer. The unbound protein (including oligomer producing endoglucanases) was washed out with the equilibrium buffer (column B, Table 1). Beta-glucosidase activity remained bound to the column and could be separately eluted with 1M NaC1.

## TABLE 1

| Enzyme | Relative Enzyme Activity (%) | |
|---|---|---|
| | A | B |
| | before ion exchange procedure | after ion exchange procedure |
| Beta-glucosidase | 100 | 0 |
| endo-1, 4, -beta-glucanase | 100 | 70 |

Endo- 1, 4- beta-glucanase and beta-glucosidase activities were measured as described by Bailey & Nevalainen (1981): Enzyme Microb. Technol. 3: 153-157. The relative enzyme activities reported in Table 1 of the Econase preparations before and after passage through an ion exchange column demonstrate the results of a typical means according to the invention preparing an essentially beta-glucosidase free preparation for use in producing the oligomeric hydrolysates contemplated by the invention.

Although Table 1 reports relative enzyme activities, the absolute amount of enzyme used in any particular example is hereafter reported in terms of the amount of enzyme activity unit of nano-katal (nkat) which stands for that amount of enzyme which produces one nanomole of reaction product in one second. (In the context of this application a hydrolysate reaction product such as an oligomer which is capable of reducing an agent such as dinitrosalicylic acid which is reduced by the hydrolysate reaction product and subsequently measured.) The method of Bailey et al., Enzyme Microb. Technol., Vol. 9, pp. 153-157 describes how such measurements of enzyme activity can be made using glucose as a standard.

Example 2 -- Cellulose Derivative Enzymatic Hydrolyses

a. Methylcellulose hydrolysate

30 g of methylcellulose (MC, Methocel MC, 64630, Fluka Chemie AG, CH-9470 Buchs, Switzerland) was mixed in 31 of water and the pH of the solution was adjusted to 5.5 with 15% phosphoric acid and the temperature was raised to 40°C. 0.3ml of the enzyme preparation having a total endo-1, 4-beta-glucanase activity of 1680 nkat from which the beta-glucosidase activity was removed chromatographically (as described in Example 1) was added to the solution. After hydrolysis for 24 hours the enzyme was inactivated by heating (90°C, 15 min.). The hydrolysate solution was subsequently cooled and freeze-dried.

The hydrolysate product contained less than 0.5% by weight of glucose and cellobiose.

The molecular weight distributions of the cellulose derivatives and their hydrolysates were determined by HPLC using a gel filtration column (TSK gel G2500PW, Toyo Soda Manufacturing Co., Ltd., Japan) with a refractive index detector (HP 1037 A) and Pharmacosmos Dextran Standards (Pharmacosmos, DK-4130, Viby Sj., Denmark). The eluent was 0.5M sodium chloride.

b. Hydroxypropylmethylcellulose Hydrolysate

20g of hydroxypropylmethylcellulose (HPMC, H-9262, Sigma Chemical Company, St. Louis, MO, U.S.A.) was mixed in 1l of water and the pH of the solution was adjusted to 5.5 with 15% phosphoric acid and the temperature was raised to 40°C. 0.24ml of the enzyme preparation having a total endo-1, 4-beta-glucanase activity of 1340 nkat from which the beta-glucosidase activity was removed chromatographically (as described in Example 1) was added to the solution. After two hours another 20g of hydroxypropylmethylcullulose was added to the solution. After the hydrolysis of 22 hours the enzyme was inactivated by heating (90°C, 15 min.). Finally the hydrolysate solution was cooled and freeze-dried.

The product contained less than 0.05% by weight of glucose and cellobiose.

c. Carboxymethylcellulose Hydrolysate

(i) Hydrolysis with Trichoderma reeseiderived enzyme preparation

20kg of carboxymethylcellulose (CMC 7MFD-type, a cellulose gum, also designated by the tradename Blanose and available from Hercules Chemical Company, 92507, Rueil-Malmaison Cedex, France; 7MFD designating a medium viscosity, food grade sodium carboxymethylcellulose having 7 out of 10 glucose units substituted with carboxymethyl) was mixed in 320l of water and the pH of the solution was adjusted to 5.5 with 15% phosphoric acid and the temperature was raised to 40°C. 0.27l of the enzyme preparation having a total endo-1, 4 beta-glucanase activity of 1,780,000 nkat from which the beta-glucosidase activity was removed chromatographically (as described in Example 1) was added to the CMC solution. After one hour another 20kg of CMC was added to the solution. After hydrolysis of 23 hours the enzyme was inactivated by heating (90°C, 15 min.). Finally, the hydrolysis solution was concentrated by conventional evaporating and spray-drying.

The product contained less than 2% by weight of glucose and cellobiose. When the same hydrolysis was carried out with the original cellulase enzyme preparation of Trichoderma reesei-fungus, the amount of produced glucose and cellobiose was above 5% by weight.

The molecular weight distribution pattern was determined as described in Example 2a.

(ii) Hydrolysis withAspergillusand Penicillium derived enzyme preparations

The enzyme preparations selected were commercially available Cellulase AP 3 (Amano Pharmaceutical Co., Ltd., Nagoya, Japan) produced using an Aspergillus strain and Cellulase CP (Sturge Enzymes, North Yorkshire, England) produced using a Penicillium strain. Carboxymethylcellulose hydrolysates were prepared as described in Example 2c(i), except that 30g of CMC-7MFD was used in 1l of water, and the amounts of enzymes added were 0.028g of Cellulase AP 3 (having a total endo-1, 4-beta-glucanase activity of 1350 nkat) and 0.048g of Cellulase CP (having a total endo-1, 4 beta-glucanase activity of 1350 knat). The viscosities and molecular weight distributions of the hydrolysates produced by either cellulase were similar to the hydrolysate produced with enzymes derived from <u>Trichoderma reesei</u>.

The viscosities of the various cellulose derivatives and their hydrolysates as described and prepared in Example 2 were measured using Haake-Rotovisco viscometer with sensor systems NV (Karlsruhe, Federal Republic of Germany) (Table 2). The viscosities were measured in water solutions at 25°C. Table 2 sets forth the concentrations (by weight) of a variety of solutions all having the same viscosity.

## TABLE 2

### Concentrations of cellulose derivatives and their respective hydrolysates in solution all having a viscosity of 20mPa.s (milli-Pascals-second) 25°C.

| Cellulose Derivatives | Concentration (by weight) |
|---|---|
| Methylcellulose | 2% |
| Methylcellulose hydrolysate | 5% |
| Hydroxypropylmethylcellulose | 3% |
| Hydroxypropylmethylcellulose hydrolysate | 10% |
| Carboxymethylcellulose | 2% |
| Carboxymethylcellulose hydrolysate | 20% |

As the data in Table 2 indicate, the hydrolysate of a cellulose derivative has a substantially lower viscosity than an equal amount by weight in aqueous solution of the cellulose derivative itself. Thus, the hydrolysate can be incorporated into a foodstuff in substantially higher quantity than the cellulose derivative itself without compromising the texture, volume, density or the like of the foodstuff.

<u>Example 3 -- The Fractionation of Carboxymethylcellulose Hydrolysate</u>

The carboxymethylcellulose hydrolysate was prepared as described in Example 2c(i), except that the raw material was CMC 7LFD (designating a low viscosity, food grade cellulose gum having 7 out of 10 glucose units substituted with carboxymethyl, designated under the tradename Blanose and available from Hercules Chemical Co., France) 1.6kg CMC was used in 8l of water and that the amount of enzyme added was 13.2 ml having a total endo-1, 4 beta-glucanase activity of 87,000 nkat. 5 ml of the hydrolysate (0.5g of dry matter) was further fractionated into three fractions by gel permeation chromatography (Pharmacia K 26/100 -column, Sephacryl S-200-gel, Pharmacia LKB Biotechnology AB, S-75182 Uppsala, Sweden). The eluent was distilled water, the flow rate was 14 ml/hour, and the fractionation process was carried out for 45 hours and fractions collected at intervals of 0.5 hours and pooled into three fractions (18 hours - 26 hours, 26 hours - 32 hours, and 32 hours - 38 hours). The molecular weight distributions of carboxymethylcellulose, carboxymethylcellulose hydrolysate, and the three further fractions were determined by HPLC as described in Example 2.

<u>Example 4 -- Chemical Hydrolysis</u>

2g of carboxymethylcellulose (Blanose Cellulose Gum 7 LFD, Hercules Chemical Co., 92507, Rueil-Malmaison Cedar, France) was hydrolyzed for one hour in 100ml of 1M sulphuric acid solution at 100°C. After hydrolysis the solution was cooled to about room temperature, neutralized to about pH 6 with 25ml of 25% (w/w) of NaOH solution and freeze-dried. This hydrolysis treatment produced a mixture of oligomers containing less than about 4% by weight of mono and disaccharides. The viscosity (and average DP) of this hydrolysate is similar to the viscosities (and average DP) of the hydrolysates produced by the enzymatic treatments described above utilizing enzymes derived from Trichoderma reesei.

The various carboxymethylcellulose hydrolysates employed as additives in the food recipes described hereinafter and referred to as EP151, EP151-2, EP151-49, EP151-51 and EP151-52 and the methods for preparing same were as follows:

a. CMC Hydrolysate EP151 was prepared as described in Example 2c(i) hereinabove.

b. CMC Hydrolysate EP151-2

20kg of carboxymethylcellulose (CMC 7LFD-type, a cellulose gum, also designated by the tradename Blanose and available from Hercules Chemical Company, 92507, Rueil-Malmaison Cedex, France, 7LFD designating a low viscosity, food grade sodium carboxymethylcellulose having 7 out of 10 glucose units substituted with carboxymethyl group) was mixed in 250l of water and the pH of the solution was adjusted to 5.8 with 15% phosphoric acid and the temperature raised to 40°C. 0.177l of the above-described Trichoderma enzyme preparation, having a total endo-1, 4 beta-glucanase activity of 1,780,000 nkat, was added to the CMC solution. After one hour another 20kg of CMC was added to the solution. After hydrolysis for 23 hours the enzyme was inactivated by heating (90°C, 15 min.). Finally, the hydrolysis solution was concentrated by spray-drying.

c. CMC Hydrolysate EP151-49

6kg of sodium carboxymethylcellulose (CMC Finnfix 5, available from Metsä-Serla, Chemical Division, SF-44100 Äänekoski, Finland, representing food grade purity and having a degree of substitution between 0.6-0.8) was mixed with 240l of water. The pH of the solution was adjusted between 5.5 and 5.9 with 15% of phosphoric acid and the temperature was maintained at 40°C. 65ml of the above-described Trichoderma enzyme preparation, the endo-$\beta$-1,4-glucanase activity of which totalled 539,000 nkat, was added to the CMC solution. After an hour another 6kg of CMC was added. After hydrolysis for 23 hours, the enzyme was inactivated by heating the solution (90°C, 15 min.). The hydrolysate was then concentrated by spray-drying.

d. CMC Hydrolysate EP151-51

6kg of sodium carboxymethylcellulose (CMC Finnfix 5) was mixed with 240l of water. Temperature and pH were as described with reference to preparation of EP151-49 (40°C, pH 5.5-5.9). 130ml of the Trichoderma enzyme preparation, the endo-$\beta$-1,4-glucanase activity of which totalled 1,079,000, was added to the CMC solution. After two hows another 6kg of CMC was added. After hydrolysis for 47 hours the enzyme was inactivated by heating the solution (90°C, 15 min.). The hydrolysate was then concentrated by evaporating and spray-drying.

e. CMC Hydrolysate EP151-52

This hydrolysate was produced as described with reference to EP151-51, except that 195ml of the enzyme preparation containing an endo-$\beta$-1,4-glucanase activity of 1,618,000 knat was used, and the hydrolysis time was 24 hours.

The viscosities, the intrinsic viscosities, the viscosity average molecular weights and the average degrees of polymerization of these various hydrolysate products are set forth in the following Table 3. The viscosities were determined using a rotational viscometer (Haake Viscotester VT 500 with sensor system NV, Karlsruhe, Federal Republic of Germany). The intrinsic viscosities were measured according to the conventional method (described in Flory, Principles of Polymer Chemistry, Cornell Univ. Press, VII-4a, Ithaca, NY (1953)) at 25°C by using a calibrated Cannon Fenske capillary viscometer (size 50, Cannon Instrument, State College, PA, USA).

The viscosity average molecular weights of the CMC hydrolysates were calculated using the Mark-Houwink equation:

$$[\eta] = KM_v^a$$

where [ $\sqrt{1}$ ] is intrinsic viscosity, $M_v$ is the average molecular weight of the polymer and K and a are hydrodynamic constants characteristic of the particular polymer-solvent system. The Mark-Houwink exponent, a, is indicative of the conformation of the polymer chain in solution. The conformation of the polymer chain in solution may be classified as an 1) impermeable dense sphere, 2) random coil, e.g. semi-permeable or free draining, and 3) rodlet or rod-like. Mark-Houwink exponents of 0.002 to about 0.5 correspond to dense spheres, exponents of about 0.5 to about 0.8 correspond to semi-permeable random coils, exponents of 0.8 to about 1.2 correspond to free draining random coils and exponents of about 1.2 to about 2 correspond to rodlets or rod-like oligomers or polymers. In an embodiment of this invention, the degradation product of the cellulose derivative comprises a mixture of oligomers of the polysaccharide having a Mark-Houwink exponent of at least 1.5 at an NaC1 concentration of about 0.005N to about 0.5N. This NaC1 concentration range is typically used when measuring Mark-Houwink exponents. The salt content of foodstuffs may also typically fall into this range. The values of K and a for CMC, which were used in this study, were: K = 0.043 in 0.2M NaC1 and a = 0.76 in 0.2M NaC1 as described in Brown and Henley, Studies on Cellulose Derivatives Part IV. The Configuration of the Polyelectrolyte Sodium Choride Solutions, Macromol. Chem., Vol. 79, pp. 68-88 (1964).

### TABLE 3

| CMC Hydrolysate | Viscosity[1] (mPas) | Intrinsic Viscosity[2] (ml/g) | Average $M_v$ | Average DP |
|---|---|---|---|---|
| 151 | 32 | 31.4 | 7400 | 39 |
| 151-2 | 20 | 22.9 | 4800 | 25 |
| 151-49 | 23 | 18.4 | 3600 | 19 |
| 151-51 | 18 | 14.0 | 2500 | 13 |
| 151-52 | 18 | 14.3 | 2600 | 13 |

1) 20% (w/w) solution, 25°C shear rate = $584s^{-1}$

2) measured in 0.2M NaCl, 25°C

It is noted that a variety of methods for determining average molecular weight exist, and therefore the values of average molecular weights determined, as well as the average DP values calculated from them, depend upon the experimental method and the basis of calculation. For example, the number average molecular weight can be determined by end group analysis, osmotic pressure, vapour pressure lowering, boiling point elevation and freezing point depression. The weight average molecular weight can be determined by light scattering experiment, the viscosity average molecular weight from the size exclusion chromatograph. All these methods can be used for determining the average DP values, although different results will be obtained depending on method and calculation used. For purposes of the present invention, the average DP were calculated from the viscosity average molecular weights, which were determined as described above (using K = 0.043 and a = 0.76 for calculation).

The $M_v$ values reported in Table 3 are first estimates obtained using the Mark-Houwink equation and the K and a values reported by Brown and Henley. However, the $M_v$ determined on the basis of the literature value a=0.74 for CMC at 0.2N NaC1 is higher than the true molecular weight of the CMC hydrolysates listed. Experimental determination of weight average molecular weight ($M_w$) by multi angle light scattering measurement shows that this conventional estimation method based on conventional literature values for k and a is not applicable to low molecular weight or relatively short chain polysaccharide derivative oligomers which are the most preferred embodiments of the invention.

CMC raw material (Mw>15,000 Daltons) has Mark-Houwink exponents of 0.83-0.97 indicating a free draining random coil conformation. In the random coil conformation, polymer coils are confined by the intra-chain interactions, therefore less change is seen in the Mark-Houwink exponent within the same range of ionic strength. However, when the weight average molecular weight is less than 15,000 Daltons, the CMC chain is not sufficiently long to form a winding coil, the polymer chain is no longer subjected to the constraint of intra-chain interactions, and a chain of free strip or rod-like configuration may form. When the ionic strength is low, the electrostatic repulsion force becomes dominant due to the negative charge of the carboxymethyl groups, and the polymer assumes its most stiff rod-like conformation with the highest value of the Mark-Houwink exponents. When the ionic strength increases, the negative charge of carboxymethyl groups is shielded, the repulsion forces between the neighboring groups are reduced, and the polymer chains relax, yielding a lower Mark-Houwink exponent.

The experimentally determined data show that the molecular weight and chain conformational characteristics of the most preferred cellulose derivative oligomeric mixtures used in the invention, i.e. mixtures comprising a significant or substantial portion of oligomers of rod-like conformation, are distinctly different from those of undegraded cellulose derivatives. As shown by the experimentally determined Mark-Houwink a values listed in Table 4 below for weight average molecular weights, $M_w$, of CMC at less than about 15,000 daltons (a=1.58 to 2.07), the literature value of a=0.74 for CMC is erroneous with respect to CMC having a $M_w$ of less than about 15,000 daltons. These experimentally determined data quantitatively indicate that relatively short chain CMC assumes a rod-like configuration as opposed to a free draining random coil conformation of the undegraded polymer.

## TABLE 4

### Mark-Houwink Equations for CMC (25°C)

| NaCl Concentration | Weight Average Molecular Weight | |
|---|---|---|
| | >15,000 | <15,000 |
| 0.005 | $[n]=0.0069M_w^{0.97}$ | $[n]=0.02 \times 10^{-5}M_w^{2.07}$ |
| 0.010 | $[n]=0.0084M_w^{0.94}$ | $[n]=0.17 \times 10^{-5}M_w^{1.82}$ |
| 0.050 | $[n]=0.0090M_w^{0.91}$ | $[n]=1.83 \times 10^{-5}M_w^{1.63}$ |
| 0.100 | $[n]=0.0116M_w^{0.88}$ | $[n]=1.18 \times 10^{-5}M_w^{1.59}$ |
| 0.200 | $[n]=0.0182M_w^{0.83}$ | $[n]=2.00 \times 10^{-5}M_w^{1.55}$ |
| 0.500 | $[n]=0.0179M_w^{0.83}$ | $[n]=1.21 \times 10^{-5}M_w^{1.58}$ |

Furthermore, the most preferred oligomeric mixtures according to the invention have a relatively narrow range of molecular weights, i.e. relatively monodispersed, having a polydispersity index ($M_w/M_n$, weight average molecular weight divided by number average molecular weight) of less than about 2.0 and typically less than about 1.8. The weight average molecular weights and number average molecular weights of a variety of CMC hydrolysate samples of different degree of hydrolysis were measured and the polydispersity index of all such hydrolysates was calculated as ranging between about 1.1 and about 1.9. Therefore, the oligomers in a most preferred mixture of oligomers extend over a relatively narrow range of $M_w$ and, even as to mixtures having an average molecular weight at or near the upper limit of $M_w$ where the oligomers may begin to assume a random coil configuration, are comprised of a significant portion, preferably a majority, of oligomers having a rod-like configuration.

In the experimental determination of $M_w$ values, CMC solutions were prepared in 0.2N NaCl solution at pH of 7. The solutions were passed through an HPLC column, and the light intensity was detected by multiangle laser light scattering using a Wyatt Technology, multiangle laser light scattering instrument, model DAWN-F. The flow rate was 0.2 ml/min. The concentrations of the solutions were detected by refractometer, and the sensitivity of the refractometer was 64. The weight average molecular weights, $M_w$, were determined using appropriate computer software.

The aforesaid degraded cellulose derivatives may be used, in accordance with the present invention, more particularly in extruded snacks, i.e. low moisture steam expanded foodstuffs having an essentially starch matrix, but which may include other ingredients for textural or flavour purposes. Snacks include many well known types of extruded or moulded foodstuff, including crisp breads, biscuits and cereal based products. The presence of the cellulose derivative in such products confers a novel glass type texture which confers crispness on the product without the need for deep fat frying or inclusion of a high fat content in the product itself. The fat content of such snacks can therefore be reduced with little or no compromise in overall quality and customer acceptability of the product. For example, snacks based on maize meal can be produced in accordance with the invention containing 0.05 to 5% by weight of the cellulose derivative, preferably 1 to 2% by weight. As compared with maize based snacks not containing the cellulose derivative, the snacks in accordance with the invention have a lighter structure, a crisp crunchy texture without frying, and less stickiness.

The cellulose derivatives may also be incorporated advantageously into gelled starch food products having a high water content, e.g. a batter, which is a starch based gel of high water content which is cooked typically by immersion in hot fat to give a product having a crisp outer surface with a moist food inside. Inclusion of the cellulose derivative leads to a reduction in the required cooking (frying) time, better water retention of the cooked foodstuff combined with a lower uptake of the cooking fat, a crisper product which retains its crispness for prolonged periods, a better appearance and improved customer acceptability. Moreover, the useful life of the cooking oil is prolonged. Preferably the batter mix contains from 1 to 5%, preferably about 2%, by weight of the cellulose derivative.

The following Examples illustrate the invention. The percentages are by weight.

Example 5

An expanded snack product based on maize meal and potato granules and containing cheese powder and flavour was produced in accordance with the following standard formulation:

| Maize meal | 72.5% |
|---|---|
| Potato granules | 10.0% |
| Oat bran | 5.0% |
| Castor sugar | 1.0% |
| Cheese powder | 8.0% |
| Cheese flavour | 2.0% |
| Salt | 1.5% |

A CMC hydrolysate produced in the manner described above, more particularly as described for hydrolysate EP151-52, was incorporated at levels from 0.05 to 5%, preferably 1 or 2%, by weight of the total formulation.

The snacks were produced by extrusion and cooked at 100°C during the extrusion process. Comparison of the products containing 1 or 2% of the cellulose derivative with those containing none of the cellulose derivative showed that the former had a lighter structure, and crisper and crunchier texture produced without frying, and less stickiness.

Example 6

A standard batter mix formulation was made from the following ingredients:

| Wheat flour | 42.0% |
|---|---|
| Water | 57.0% |
| Salt | 1.0% |

These ingredients were mixed together and whisked to produce a uniform batter. The cellulose derivative (as in Example 5) was incorporated at a level of 1, 2 or 5% by weight to replace the corresponding amount of the flour.

The batter was used to coat mushrooms which were then fried at 170°C. It was found that the standard frying time of 10 minutes (in the absence of the cellulose derivative) was reduced to 8 minutes by the presence of 1% cellulose derivative, to 6 minutes by the presence of 2% cellulose derivative, and to 4 minutes by 5% of the cellulose derivative.

The fried mushrooms produced with the batter in accordance with the present invention showed greater water retention and hence higher weight and lower fat uptake. They maintained their crispness for longer and had improved appearance and customer acceptability. Moreover, the oil used for the frying had a longer life.

## Claims

1. A gelled starch food product comprising from 0.05 to 10 % by weight of the starch of a mixture of oligomers derived from the degradation of a cellulose derivative, the said mixture of oligomers having an average degree of polymerization in the range of 3 to 500 and an average molecular weight in the range of 500 to 100,000 determined on the basis of intrinsic viscosity.

2. A gelled starch food product according to claim 1 wherein the cellulose derivative is carboxymethylcellulose, methyl-cellulose, methylethylcellulose, hydroxypropylcellulose, hydroxypropylmethylcellulose, or a mixture thereof.

3. A gelled starch food product according to claim 1 or 2 wherein the mixture of oligomers has an average degree of polymerization in the range of 5 to 100.

4. A gelled starch food product according to any one of claims 1 to 3 wherein the mixture of oligomers has an average molecular weight less than 15,000 daltons, a polydispersity less than 2, and contains less than 25% by weight of monosaccharides and disaccharides.

5. A gelled starch food product according to any one of claims 1 to 4 which is cooked at elevated temperature during extrusion or by frying.

6. A gelled starch food product according to any one of claims 1 to 5 in the form of a snack based on maize meat containing 0.05 to 5 % by weight of the mixture of oligomers.

7. A gelled starch food product according to any one of claims 1 to 5 in the form of a cooked batter containing from 1 to 5 % by weight of the said mixture of oligomers.

8. A gelled starch food product according to any one of the preceding claims wherein the mixture of oligomers is produced by hydrolysing the cellulose derivative with a cellulase enzyme preparation.

9. A gelled starched food product according to any one of claims 1 to 7 wherein the mixture of oligomers is produced by chemical hydrolysis or physical degradation of the cellulose derivative.

## Patentansprüche

1. Geliertes Stärkelebensmittelprodukt mit von 0,05 bis 10 Gewichtsprozent Stärke einer Mischung aus Oligomeren, die aus einem Abbau eines Celluslosederivates abgeleitet sind, wobei die Mischung von Oligomeren einen mittleren Polymerisationsgrad im Bereich von 3 bis 500 und ein mittleres Molekulargewicht im Bereich von 500 bis 100.000 aufweist, was auf der Basis der intrinsischen Viskosität bestimmt ist.

2. Geliertes Stärkelebensmittelprodukt nach Anspruch 1, bei dem das Cellulosederivat Carboxymethylcellulose, Methylcellulose, Methylethylcellulose, Hydroxypropylcellulose, Hydroxypropylmethylcellulose oder eine Mischung davon ist.

3. Geliertes Stärkelebensmittelprodukt nach Anspruch 1 oder 2, bei dem die Mischung von Oligomeren einen mittleren Polymerisationsgrad in dem Bereich von 5 bis 100 aufweist.

4. Geliertes Stärkelebensmittelprodukt nach einem der Ansprüche 1 bis 3, bei dem Mischung von Oligomeren ein mittleres Molikular-gewicht von weniger als 15.000 Dalton, eine Polydispersitie von weniger als 2 aufweist und weniger als 25 Gewichtsprozent Monosaccharide und Disaccharide enthält.

5. Geliertes Stärkelebensmittelprodukt nach einem der Ansprüche 1 bis 4, das bei erhöhter Temperatur während Extrudieren oder durch Braten gekocht wird.

6. Geliertes Stärkelebensmittelprodukt nach einem der Ansprüche 1 bis 5 in der Form eines Snacks auf der Grundlage von Maismehl mit 0,05 bis 5 Gewichtsprozent der Mischung von Oligomeren.

7. Geliertes Stärkelebensmittelprodukt nach einem der Ansprüche 1 bis 5 in der Form eines gekochten Teiges, der von 1 bis 5 Gewichtsprozent der Mischung von Oligomeren enthält.

8. Geliertes Stärkelebensmittelprodukt nach einem der vorhergehenden Ansprüche, bei dem die Mischung von Oligomeren erzeugt wird durch Hydrolyse des Cellulosederivates mit einer Cellulaseenzymdarstellung.

9. Geliertes Stärkelebensmittelprodukt nach einem der Ansprüche 1 bis 7, bei dem die Mischung von Oligomeren durch chemische Hydrolyse oder physikalisches Zersetzen des Cellulosederivates erzeugt wird.


**Revendications**

1. Produit alimentaire à base d'amidon gélifié comprenant de 0,05 à 10 % en poids de l'amidon d'un mélange d'oligomères dérivés de la dégradation d'un dérivé de la cellulose, ledit mélange d'oligomères ayant un degré moyen de polymérisation dans la gamme de 3 à 500 et un poids moléculaire moyen dans la gamme de 500 à 100 000 déterminé à partir de la viscosité intrinsèque.

2. Produit alimentaire à base d'amidon gélifié selon la revendication 1, dans lequel le dérivé de la cellulose est la carboxyméthylcellulose, la méthylcellulose, la méthyléthylcellulose, l'hydroxypropylcellulose, l'hydroxypropylméthylcellulose ou un de leurs mélanges.

3. Produit alimentaire à base d'amidon gélifié selon la revendication 1 ou 2, dans lequel le mélange d'oligomères a un degré moyen de polymérisation dans la gamme de 5 à 100.

4. Produit alimentaire à base d'amidon gélifié selon l'une quelconque des revendications 1 à 3, dans lequel le mélange d'oligomères a un poids moléculaire moyen inférieur à 15 000 daltons, une polydispersité inférieure à 2 et contient moins de 25 % en poids de monosaccharides et de disaccharides.

5. Produit alimentaire à base d'amidon gélifié selon l'une quelconque des revendications 1 à 4, qui est cuit à une température élevée pendant l'extrusion ou par friture.

6. Produit alimentaire à base d'amidon gélifié selon l'une quelconque des revendications 1 à 5 sous forme d'un amuse-gueule à base de farine de maïs contenant 0,05 à 5 % en poids du mélange d'oligomères.

7. Produit alimentaire à base d'amidon gélifié selon l'une quelconque des revendication 1 à 5 sous forme d'une pâte cuite contenant de 1 à 5 % en poids dudit mélange d'oligomères.

8. Produit alimentaire à base d'amidon gélifié selon l'une quelconque des revendications précédentes, dans lequel le mélange d'oligomères est produit par hydrolyse du dérivé de la cellulose avec une préparation d'enzyme cellulase.

9. Produit alimentaire à base d'amidon gélifié selon l'une quelconque des revendications 1 à 7, dans lequel le mélange d'oligomères est produit par hydrolyse chimique ou dégradation physique du dérivé de la cellulose.